Europäisches Patentamt

European Patent Office

Office européen des brevets

(11) Veröffentlichungsnummer: **0 297 438**
**A1**

## (12) EUROPÄISCHE PATENTANMELDUNG

(21) Anmeldenummer: 88110050.7

(22) Anmeldetag: 23.06.88

(51) Int. Cl.4: **A61B 5/05**

(30) Priorität: 23.06.87 DE 3720662

(43) Veröffentlichungstag der Anmeldung:
04.01.89 Patentblatt 89/01

(84) Benannte Vertragsstaaten:
**AT CH DE FR GB IT LI**

(71) Anmelder: **Glamann, Edith-Ingrid**
**Bahnstrasse 2a**
**D-6231 Sulzbach/Ts(DE)**

(72) Erfinder: **Glamann, Edith-Ingrid**
**Bahnstrasse 2a**
**D-6231 Sulzbach/Ts(DE)**

(74) Vertreter: **Patentanwälte Kirschner & Grosse**
**Forstenrieder Allee 59**
**D-8000 München 71(DE)**

(54) **Hochfrequenz-Diagnosegerät.**

(57) Bei einem Hochfrequenz-Diagnosegerät zur Erfassung von krankem Gewebe in einem menschlichen oder tierischen Körper ist zur Durchführung von Medikamententests eine Aufnahmeeinrichtung (20) für Medikamente in dem Meßkreis angeordnet, der von der Hochfrequenz-Energiequelle über die Sendeelektrode (6) und die Tastelektrode (10, 12) zu der Meßvorrichtung (2) führt. Die Aufnahmeeinrichtung, beispielsweise ein Behälter oder eine Medikamentenwabe, sind an der Sendeelektrode angeordnet. Die Tastelektrode hat eine auf die Andruckkraft der Tastelektrode ansprechende Kraftmeßeinrichtung (32, 46) und einen damit verbundenen Signalgeber (42, 40), der bei einem vorgegebenen Wert der Andruckkraft ein Signal abgibt, wobei die Messung bei gleichbleibender Andruckkraft der Tastelektrode durchführbar ist.

Fig: 1

EP 0 297 438 A1

Die Erfindung betrifft ein Hochfrequenz-Diagnosegerät zur Erfassung von krankem Gewebe in einem menschlichen oder tierischen Körper mit einer Hochfrequenz-Energiequelle, einer mit der Hochfrequenz-Energiequelle verbundenen Sendeelektrode, die ein Hochfrequenzfeld abstrahlt, einer Tastelektrode, die das Hochfrequenzfeld nach Durchgang durch den Körper erfaßt, und einer mit der Tastelektrode verbundenen Meßvorrichtung zwischen der Tastelektrode und der Hochfrequenz-Energiequelle.

Mit einem derartigen Hochfrequenz-Diagnosegerät (Hochfrequenz im folgenden: HF) kann krankes Gewebe in einem menschlichen oder tierischen Körper erfaßt werden, da gesundes Gewebe einen anderen Einfluß auf das HF-Feld hat als krankes Gewebe. Man eicht zunächst das Gerät an gesundem Gewebe und kann dann negative und positive Abweichungen von dem Eichwert feststellen, wobei die Abweichungen auf bestimmte Krankheitsbilder hinweisen. Die verwendeten Frequenzen liegen zwischen etwa 250 kHz und 750 kHz, wobei ein üblicher Wert 350 kHz mit 1200 Hz amplitudenmoduliert ist.

Der Erfindung liegt die Aufgabe zugrunde, dieses Gerät nicht nur für die Diagnose sondern auch für den Medikamententest auszugestalten, bei dem ein für den festgestellten Krankheitsbefund geeignetes Medikament ausgesucht wird.

Dazu ist das erfindungsgemäße HF-Diagnosegerät der eingangs genannten Art dadurch gekennzeichnet, daß eine Aufnahmevorrichtung für Medikamente in dem Meßkreis, der von der Hf-Energiequelle über die Sendeelektrode und die Tastelektrode zu der Meßvorrichtung führt, angeordnet ist. Die in die Aufnahmeeinrichtung eingebrachten Medikamente beeinflussen die Messung derart, daß ein bei einem kranken Gewebe festgestellter Befund, der zu einer Abweichung der Anzeige von dem Wert für gesundes Gewebe führt, verändert wird. Wenn die durch das Medikament herbeigeführte Änderung des Ausschlags am Meßgerät die Abweichung des Ausschlags von dem geeichten Ausschlag bei gesundem Gewebe aufhebt, ist das richtige Medikament gefunden.

Nach einer vorteilhaften Ausgestaltung der Erfindung ist eine Sendeelektrode für ein HF-Diagnosegerät dadurch gekennzeichnet, daß die Aufnahmeeinrichtung an der Sendeelektrode angeordnet ist. Dies führt zu einer einfachen Anordnung und einer wirksamen Erfassung des Medikaments bei der Messung.

Die Aufnahmeeinrichtung kann ein Behälter für Medikamente oder eine Medikamentenwabe aufweisen, wobei sowohl flüssige als auch feste und in einer Ampulle befindliche Medikamente oder Mischungen aus Medikamenten getestet werden können.

Nach einer vorteilhaften Ausgestaltung der Erfindung ist eine Tastelektrode insbesondere für ein HF-Diagnosegerät dadurch gekennzeichnet, daß die Tastelektrode (10, 12) eine Kraftmeßeinrichtung (32 bzw. 46) aufweist, die die Andruckkraft der Tastelektrode auf den Körper mißt.

In der Praxis hat es sich gezeigt, daß der über die Tastelektrode erfaßte Wert der Feldstärke durch unterschiedlichen Druck mit der Tastelektrode auf den Körper unterschiedlich ausfallen kann. Daher muß dafür gesorgt werden, daß die Tastelektrode immer mit dem gleichen Druck gegen den Körper gedrückt wird, um dieser Fehlermöglichkeit auszuschalten. Mit der Kraftmeßeinrichtung und dem damit verbundenen Signalgeber ist es nun möglich, die Messung immer bei der gleichen Andruckkraft vorzunehmen, so daß die genannte Fehlermöglichkeit ausgeschaltet wird. Diese Abtastelektrode ist besonders in Zusammenhang mit dem Medikamententest vorteilhaft, weil es beim Medikamententest auf den Vergleich mehrerer Feldstärken ankommt, nämlich der Feldstärke bei gesundem Gewebe, der Feld stärke bei krankem Gewebe und der Feldstärke bei Kompensation des Krankheitseffekts durch ein Medikament. Bei der reinen Diagnose kommt es dagegen nur auf die Abweichung der Feldstärken zwischen gesundem und krankem Gewebe an. Dennoch ist auch bei der Diagnose die Verwendung der oben angegebenen Tastelektrode vorteilhaft.

Nach einer weiteren vorteilhaften Ausgestaltung der Erfindung ist die Tastelektrode dadurch gekennzeichnet, daß die Kraftmeßeinrichtung ein piezoelektrisches Meßelement aufweist. Bei dieser Ausgestaltung sind nur geringe Bewegungen der Teile der Elektrode gegeneinander erforderlich und die piezoelektrische Messung ist sehr genau, so daß die Kraft, mit der die Tastelektrode gegen den Körper gedrückt wird, genau festgestellt und auf einen genauen Wert genormt werden kann.

Nach einer weiteren Ausgestaltung der Erfindung ist die Tastelektrode dadurch gekennzeichnet, daß die Kraftmeßeinrichtung eine Feder, die zwischen einem Elektrodenrohr der Tastelektrode und einer daran verschiebbar gelagerten Elektrodenspitze angeordnet ist, aufweist.

Eine weitere vorteilhafte Ausgestaltung der zuletzt genannten Tastelektrode ist dadurch gekennzeichnet, daß die Elektrodenspitze auf einem in dem Elektrodenrohr geführten Zapfen angeordnet ist, der einen Kragen aufweist, der ein Widerlager für das eine Ende einer Druckfeder bildet, deren anderes Ende an einem Lagereinsatz in dem Elektrodenrohr abgestützt ist. Durch die Verwendung der Druckfeder ergibt sich eine besonders einfache mechanische Anordnung für die Kraftmeßeinrichtung.

Nach einer weiteren Ausgestaltung der Erfindung ist die Tastelektrode dadurch gekennzeichnet, daß mit der Kraftmeßeinrichtung ein Signalgeber verbunden ist, der bei einem vorgegebenen Wert der Andruckkraft ein Signal abgibt. Durch den Signalgeber, der ein optisches oder akustisches Signal abgeben kann, kann auf besonders einfache Weise festgestellt werden, daß die Kraftmeßeinrichtung mit der vorgegebenen Kraft beaufschlagt wird, bei der die Messung durchgeführt werden soll.

Bei der mechanischen Ausführung der Kraftmeßeinrichtung ist es vorteilhaft, wenn die Tastelektrode dadurch gekennzeichnet ist, daß der Signalgeber einen an dem Zapfen angeordneten, elektrisch leitenden Abschnitt aufweist, über den zwei damit in Kontakt stehende Kontakte miteinander elektrisch kurzschließbar sind. Diese Lösung stellt eine einfache Integration des Signalgebers in die mechanische Kraftmeßeinrichtung dar.

Ausführungsbeispiele der Erfindung werden nun anhand der beiliegenden Zeichnungen beschrieben. Es zeigen:

Fig. 1 eine schematische Darstellung eines HF-Diagnosegerätes;

Fig. 2 eine teilweise geschnittene Darstellung einer Tastelektrode; und

Fig. 3 ein anderes Ausführungsbeispiel einer Tastelektrode.

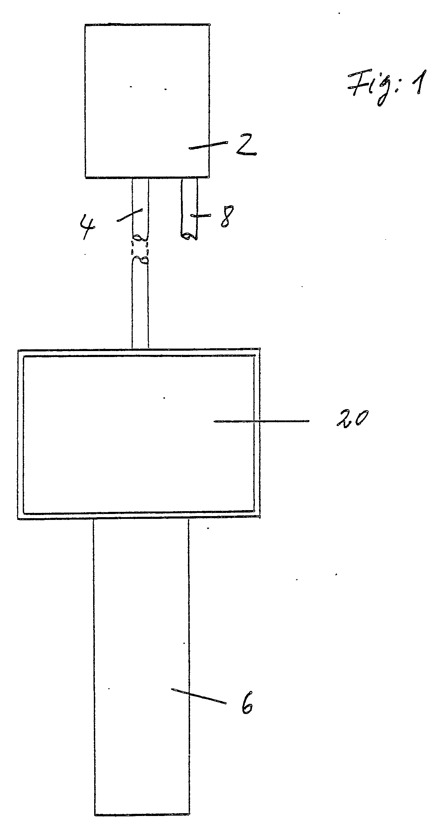

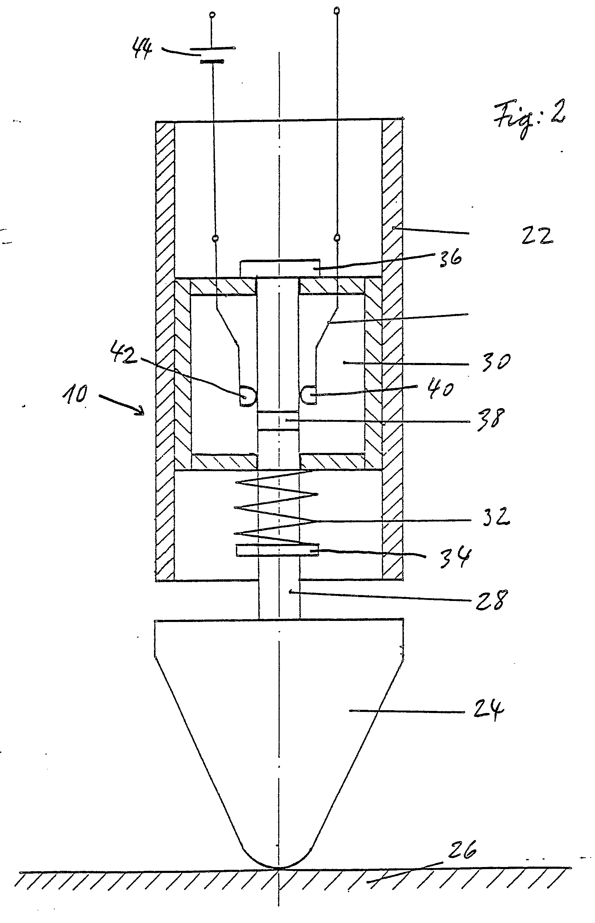

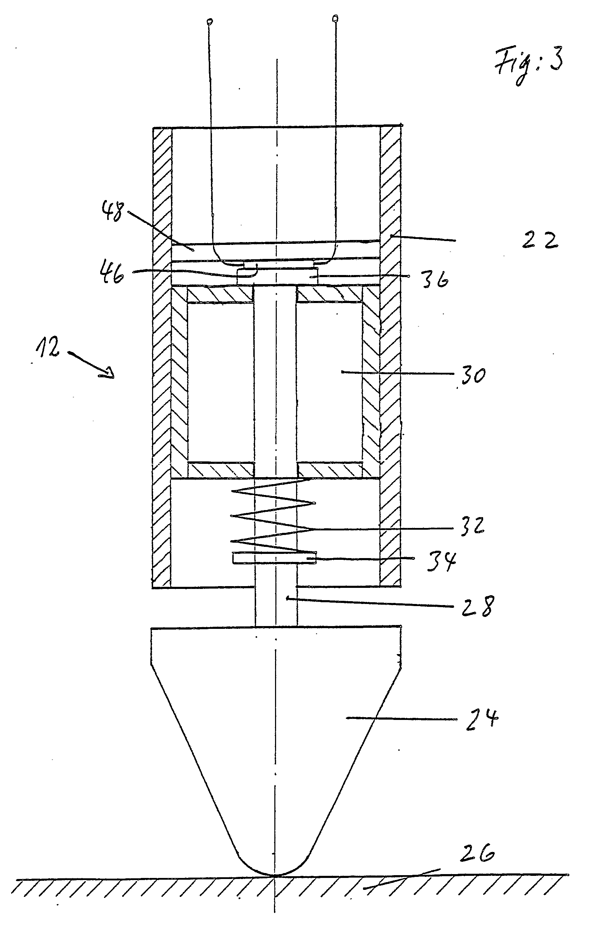

In Fig. 1 ist schematisch ein Gehäuse 2 dargestellt, welches eine HF-Energiequelle, eine Meßvorrichtung zur Messung der Feldstärke eines HF-Feldes und eine Anzeigeeinrichtung, beispielsweise eine Skala, enthält. Die HF-Energiequelle in dem Gehäuse 2 ist über eine Leitung 4 mit einer Sendeelektrode 6 verbunden. Die Meßvorrichtung in dem Gehäuse 2 mit einer Leitung 8 mit einer Tastelektrode 10 (Fig. 2) bzw. 12 (Fig. 3) verbunden. Die in der HF-Energiequelle erzeugte Energie wird über die Sendeelektrode 6 als elektrisches Feld abgestrahlt, das den zu diagnostizierenden Körper durchdringt und von der Tastelektrode 10 abgetastet wird. Die von der Tastelektrode 10, 12 gemessene Feldstärke wird in der zwischen der Tastelektrode und der HF-Energiequelle liegenden Meßvorrichtung in dem Gehäuse 2 gemessen und an der Skala angezeigt.

Die Anzeige ist daher ein Maß für die Beeinflussung des von der Sendeelektrode 6 abgestrahlten HF-Feldes durch den zu diagnostizierenden Körper. Das HF-Feld wird durch gesundes und krankhaftes Gewebe in dem Körper unterschiedlich beeinflußt, so daß nach Eichung auf gesundes Gewebe das Diagnosegerät krankhaftes Gewebe feststellen kann.

Nach Feststellung und Lokalisierung von krankem Gewebe im Körper soll mit dem Diagnosegerät auch festgestellt werden, welches Medikament für die Heilung des krankhaften Gewebes geeignet ist. Dazu ist eine Aufnahmeeinrichtung 20 für Medikamente in dem Meßkreis, der von der HF-Energiequelle über die Sendeelektrode 6 und die Tastelektrode 10 bzw. 12 zu der Meßeinrichtung führt, angeordnet. Bei dem gezeigten Ausführungsbeispiel in Fig. 1 ist die Aufnahmeeinrichtung 20 an der Sendeelektrode 6 angeordnet, so daß die HF-Energie über die Leitung 4 und die Aufnahmeeinrichtung 20 an die Sendeelektrode 6 gelangt und dadurch von dem in der Aufnahmeeinrichtung 20 enthaltenen Medikament beeinflußt wird. Die Aufnahmeeinrichtung 20 kann - wie schematisch dargestellt - ein Behälter für Medikamente oder eine sog. Medikmentenwabe sein, wobei lediglich sichergestellt sein muß, daß das von der Sendeelektrode 6 abgestrahlte HF-Feld durch das in der Aufnahmeeinrichtung 20 enthaltene Medikament beeinflußt wird.

Mit der bisher beschriebenen Anordnung werden die Diagnose und Lokalisierung einer krankhaften Stelle am Körper und der Medikamententest wie folgt durchgeführt. Der Körper wird mit dem HF-Feld überlagert, und das Gerät wird an einer Stelle mit gesundem Gewebe auf einen Mittelwert geeicht, wobei ein gesundes Gewebe durch einen homogenen Feldlinienverlauf charakterisiert ist. Ein gebeugter Feldlinienverlauf, verursacht durch eine elektrische Inhomogenität im Körper, gibt einen diagnostischen Hinweis auf krankes Gewebe. Bei einer topographisch lokalisierten, kranken Stelle, die durch einen von dem Mittelwert abweichenden Zeigerstand an der Skala erkennbar ist, wird ein Medikamententest durchgeführt. Dazu wird ein der lokalisierten Krankheit zugeordnetes Medikament in die Aufnahmeeinrichtung 20 eingeführt. Aufgrund der Beeinflussung des HF-Feldes durch das Medikament verändert sich der Feldlinienverlauf, was wiederum durch einen entsprechenden Ausschlag an der Skala erkennbar ist. Wenn das der lokalisierten Krankheit korrekt zugeordnete Medikament, ein Arzneimittel oder eine Arzneimittelkombination, in die Aufnahmeeinrichtung 20 gegeben wird, wird der Ausschlag an der Skala auf den Mittelwert zurückgeführt.

Beispielsweise wird eine entzündliche Lebererkrankung an einem Patienten durch einen über den Mittelwert hinausgehenden und eine degenerative Lebererkrankung durch einen unter den Mittelwert absinkenden Ausschlag an dem Anzeigegerät angezeigt. Wird dann im Falle eines zu hohen Ausschlages ein entzündungshemmendes Medikament in die Aufnahmeeinrichtung gegeben, kehrt der Ausschlag an dem Anzeigegerät in Richtung auf den Mittelwert zurück.

In Fig. 2 ist ein Schnitt durch ein Ausführungsbeispiel einer Tastelektrode 10 gezeigt, die ein Elektrodenrohr 22 und eine Elektrodenspitze 24 aus isolierendem Material aufweist. Die Elektrodenspitze 24, mit der die Tastelektrode 10 auf eine Körperoberfläche 26 aufgesetzt wird, hat einen Zapfen 28, mit dem die Elektrodenspitze 24 in einem Lagereinsatz 30 gelagert ist, der in dem Elektrodenrohr 22 befestigt ist. Eine Druckfeder 32 ist zwischen einem Kragen 34 an dem Zapfen 28 und dem Lagereinsatz 30 angeordnet und drückt die Elektrodenspitze 24 nach unten (Blickrichtung wie in Fig. 2). Die ausgefahrene Endstellung der Elektrodenspitze 24 wird durch einen Endansatz 36 an dem Zapfen 28 fixiert, wobei der Ansatz 36 in der Endstellung an dem Lagereinsatz 30 ansteht. Der Zapfen 28 hat einen elektrisch leitfähigen Abschnitt 38, beispielsweise einen Metallring, über den zwei damit in Kontakt stehende Kontakte 40, 42 miteinander elektrisch kurzzuschließen sind, wenn die Elektrodenspitze 24 gegen die Kraft der Feder 32 soweit nach oben gedrückt wird, daß der Abschnitt 38 in Kontakt mit den Kontakten 40, 42 kommt. Ein Kurzschluß zwischen den Kontakten 40, 42 wird durch einen Signalgeber mit einer Stromquelle 44 als optisches oder akustisches Signal angezeigt. Wenn ein Signal gegeben wird, ist daher sichergestellt, daß die Elektrodenspitze 24 mit einer durch die Federkraft vorgegebenen Kraft auf die Körperoberfläche 26 gedrückt wird, so daß die Messungen mit immer der gleichen Andruckkraft durchgeführt werden und Meßfehler ausgeschaltet werden können, die dadurch verursacht werden, daß Messungen mit unterschiedlichen Andruckkräften durchgeführt werden.

Fig. 3 zeigt ein anderes Ausführungsbeispiel einer Tastelektrode 12, bei der die Messung der Andruckkraft auf piezoelektrischem Wege erfolgt. In Fig. 3 tragen die Teile, die auch in Fig. 2 gezeigt sind, dieselben Bezugszeichen. Auf der Oberseite (Blickrichtung wie in Fig. 3) des Anschlags 36 ist ein Piezoelement 46 angeordnet, das auf einer Wand 48 aufliegt, die in dem Elektrodenrohr 22 befestigt ist. Bei einem Druck auf die Elektrodenspitze 24 wird ein entsprechender Wert der Auflagekraft an dem Piezoelement 46 gemessen. Die an das Piezoelement 46 angeschlossene Meßschaltung gibt bei einem bestimmten vorgegebenen Wert der gemessenen Kraft wiederum ein optisches oder akustisches Signal. Die Feder 32 dient in diesem Fall lediglich zur Entlastung des Piezoelements 46, so daß durch Abstimmung der Feder 32 auf das jeweilige Piezoelement das Piezoelement in seinem optimalen Arbeitsbereich eingesetzt werden kann.

Die Tastelektroden 10, 12 in den Fig. 2 bzw. 3 weisen demnach eine auf die Andruckkraft der Tastelektrode ansprechende Kraftmessungseinrichtung und einen damit verbundenen Signalgeber auf, der bei einem vorgegebenen Wert der Andruckkraft ein Signal abgibt. Eine solche Kraftmeßeinrichtung kann auch als induktive oder kapazitive Einrichtung ausgebildet sein, wobei jedoch die oben beschriebenen Ausführungsbeispiele bevorzugt sind.

## Ansprüche

1. Hochfrequenz-Diagnosegerät zur Erfassung von krankem Gewebe in einem menschlichen oder tierischen Körper mit einer Hochfrequenz-Energiequelle, einer mit der Hochfrequenz-Energiequelle verbundenen Sendeelektrode, die ein Hochfrequenzfeld abstrahlt, einer Tastelektrode, die das Hochfrequenzfeld nach Durchgang durch den Körper erfaßt, und mit einer mit der Tastelektrode verbundenen Meßeinrichtung zwischen der Tastelektrode und der Hochfrequenz-Energiequelle, **dadurch gekennzeichnet,** daß eine Aufnahmeeinrichtung (20) für Medikamente in dem Meßkreis, der von der Hochfrequenz-Energiequelle über die Sendeelektrode und die Tastelektrode zu der Meßvorrichtung führt, angeordnet ist.

2. Sendeelektrode für ein Hochfrequenz-Diagnosegerät nach Anspruch 1, **dadurch gekennzeichnet,** daß die Aufnahmeeinrichtung (20) an der Sendeelektrode (6) angeordnet ist.

3. Sendeelektrode nach Anspruch 2, **dadurch gekennzeichnet,** daß die Aufnahmeeinrichtung ein Behälter für Medikamente ist.

4. Sendeelektrode nach Anspruch 2, **dadurch gekennzeichnet,** daß die Aufnahmeeinrichtung eine Medikamentenwabe aufweist.

5. Tastelektrode für ein Hochfrequenz-Diagnosegerät zur Erfassung von krankem Gewebe in einem menschlichen oder tierischen Körper mit einer Hochfrequenz-Energiequelle, einer mit der Hochfrequenz-Energiequelle verbundenen Sendeelektrode, die ein Hochfrequenzfeld abstrahlt, einer Tastelektrode, die an den Körper gedrückt wird und das Hochfrequenzfeld nach Durchgang durch den Körper erfaßt, und einer mit der Tastelektrode verbundenen Meßvorrichtung zwischen der Tastelektrode und der Hochfrequenz-Energiequelle, **dadurch gekennzeichnet,** daß die Tastelektrode (10, 12) eine Kraftmeßeinrichtung (32 bzw. 46) aufweist, die die Andruckkraft der Tastelektrode auf den Körper mißt.

6. Tastelektrode nach Anspruch 5, **dadurch gekennzeichnet,** daß die Kraftmeßeinrichtung ein piezoelektrisches Meßelement (46) aufweist.

7. Tastelektrode nach Anspruch 5, **dadurch gekennzeichnet,** daß die Kraftmeßeinrichtung eine Feder (32), die zwischen einem Elektrodenrohr (22) der Tastelektrode (10, 12) und einer daran verschiebbar gelagerten Elektrodenspitze (24) angeordnet ist, aufweist.

8. Tastelektrode nach Anspruch 7, **dadurch gekennzeichnet,** daß die Elektrodenspitze (24) auf einem in dem Elektrodenrohr (22) geführten Zapfen (28) angeordnet ist, der einen Kragen (34) aufweist, der ein Widerlager für das eine Ende einer Druckfeder bildet, deren anderes Ende an einem Lagereinsatz in dem Elektrodenrohr abgestützt ist.

9. Tastelektrode nach einem der Ansprüche 5 bis 8, **dadurch gekennzeichnet,** daß mit der Kraftmeßeinrichtung (32 bzw. 46) ein Signalgeber verbunden ist, der bei einem vorgegebenen Wert der Andruckkraft ein Signal abgibt.

10. Tastelektrode nach Anspruch 8 und 9, **dadurch gekennzeichnet,** daß der Signalgeber einen an dem Zapfen (28) angeordneten, elektrisch leitenden Abschnitt (38) aufweist, über den zwei damit in Kontakt stehende Kontakte (40, 42) mitei- nander elektrisch kurzschließbar sind.

Fig: 1

2

4

8

20

6

Fig. 2

Fig: 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-C- 950 402 (L. MACHTS)<br>* Seite 1, Zeile 24 - Seite 3, Zeile 23; Figuren I,II,III *<br>--- | 1,5,7 | A 61 B 5/05 |
| A | DE-A-2 525 621 (F. MORELL)<br>* Seite 3, Zeilen 1-13; Figur 1 *<br>--- | 1-4 | |
| A | CH-A- 352 781 (L. MACHTS)<br>* Seite 1, Zeilen 21-47; Seite 2, Zeile 37 - Seite 3, Zeile 7; Figur 1 *<br>----- | 1,5 | |

**RECHERCHIERTE SACHGEBIETE (Int. Cl.4)**

A 61 B 5/00

Der vorliegende Recherchenbericht wurde für alle Patentansprüche erstellt

| Recherchenort | Abschlußdatum der Recherche | Prüfer |
|---|---|---|
| BERLIN | 01-09-1988 | WEIHS J.A. |